# EUROPÄISCHE PATENTSCHRIFT

(11) **EP 1 313 431 B1**
(45) Veröffentlichungstag und Bekanntmachung des Hinweises auf die Patenterteilung: **15.03.2006**
(21) Anmeldenummer: 01962950.0
(22) Anmeldetag: 16.08.2001
(51) Int. Cl.: A61K 8/91, A61Q 5/00, C08F 283/06, C08F 290/14, C08F 283/12

(54) **HAARKOSMETISCHE FORMULIERUNGEN**
HAIR COSMETIC FORMULATIONS
FORMULATIONS COSMETIQUES POUR CHEVEUX

(30) Priorität: 21.08.2000 DE 10041163
(43) Veröffentlichungstag der Anmeldung: 28.05.2003
(73) Patentinhaber: BASF Aktiengesellschaft, 67056 Ludwigshafen (DE)
(72) Erfinder: GOTSCHE, Michael, 68167 Mannheim (DE); WOOD, Claudia, 69469 Weinheim (DE)
(86) Internationale Anmeldenummer: PCT/EP2001/009437
(87) Internationale Veröffentlichungsnummer: WO 2002/015853

(56) Entgegenhaltungen:
- EP-A- 0 635 526
- EP-A- 0 769 290
- WO-A-00/49998
- WO-A-94/08554
- WO-A-99/04750
- US-A- 3 188 275
- DATABASE WPI Section Ch, Week 200040 Derwent Publications Ltd., London, GB; Class A14, AN 2000-454349 XP002179769 & JP 2000 086468 A (SHISEIDO CO LTD), 28. März 2000 (2000-03-28)
- DATABASE WPI Section Ch, Week 199953 Derwent Publications Ltd., London, GB; Class A28, AN 1999-615599 XP002179770 & JP 11 269239 A (NIPPON SHOKUBAI CO LTD), 5. Oktober 1999 (1999-10-05)

## Beschreibung

Die vorliegende Erfindung betrifft wäßrige oder wäßrig/alkoholische haarkosmetische Formulierungen enthaltend als Filmbildner Polymerisate, hergestellt durch Polymerisation von Vinylestern und optional weiteren radikalisch copolymerisierbaren Monomeren in Gegenwart einer polyetherhaltigen Verbindung.

Für die Festigung von Haarfrisuren werden seit fast 50 Jahren synthetische Polymere eingesetzt. Während in der Anfangszeit bevorzugt Vinyllactam-Homo- und Copolymere zum Einsatz kamen, haben später carboxylatgruppenhaltige Polymere zunehmend an Bedeutung gewonnen. Anforderungen an Haarfestigerharze sind z.B. eine starke Festigung bei hoher Luftfeuchtigkeit, Elastizität, Auswaschbarkeit vom Haar und Verträglichkeit mit weiteren Formulierungskomponenten. Schwierigkeiten bereitet die Kombination verschiedener Eigenschaften. So zeigen Polymere mit guten Festigungseigenschaften oftmals geringe Elastizitäten, so daß bei mechanischer Beanspruchung der Frisur die Festigungswirkung durch Schädigung des Polymerfilm oft erheblich beeinträchtigt wird.

Verbesserungsbedarf besteht daher vor allem bei der Erzeugung elastischer Frisuren bei gleichzeitig starker Festigung auch bei hoher Luftfeuchtigkeit, guter Auswaschbarkeit und gutem Griff des Haares.

Ziel der Erfindung war es daher, haarkosmetische Formulierungen mit filmbildenden Polymeren zu finden, die der Frisur eine starke Festigung bei gleichzeitig hoher Elastizität verleihen.

Die Aufgabe wurde erfindungsgemäß gelöst durch Verwendung von Polymerisaten, die erhältlich sind durch radikalische Polymerisation von
a) mindestens einem Vinylester in Gegenwart von
b) polyetherhaltigen Verbindungen

und gegebenenfalls mindestens eines weiteren copolymerisierbaren Monomeren c)
in haarkosmetischen Formulierungen.

Pfropfpolymerisate von Polyvinylalkohol auf Polyalkylenglykole sind bereits bekannt.

DE 1 077 430 beschreibt ein Verfahren zur Herstellung von Pfropfpolymerisaten von Vinylestern auf Polyalkylenglykole.

DE 1 094 457 und DE 1 081 229 beschreiben Verfahren zur Herstellung von Pfropfpolymerisaten von Polyvinylalkohol auf Polyalkylenglykolen durch Verseifung der Vinylester und deren Verwendung als Schutzkolloide, wasserlösliche Verpackungsfolien, als Schlichte- und Appreturmittel für Textilien und in der Kosmetik.

Bei der Herstellung der erfindungsgemäß verwendeten Polymerisate kann es während der Polymerisation zu einer Pfropfung auf die polyetherhaltigen Verbindungen (b) kommen, was zu den vorteilhaften Eigenschaften der Polymerisate führen kann. Es sind jedoch auch andere Mechanismen als Pfropfung vorstellbar.

Je nach Pfropfungsgrad sind unter den erfindungsgemäß verwendeten Polymerisaten sowohl reine Pfropfpolymerisate als auch Mischungen der o.g. Pfropfpolymerisate mit ungepfropften polyetherhaltigen Verbindungen und Homo- oder Copolymerisaten der Monomeren a) und c) zu verstehen.

Als polyetherhaltige Verbindungen b) können Umsetzungsprodukte von Polyethyleniminen mit Alkylenoxiden eingesetzt werden. Als Alkylenoxide werden in diesem Fall bevorzugt Ethylenoxid, Propylenoxid, Butylenoxid und Mischungen aus diesen, besonders bevorzugt Ethylenoxid verwendet. Als Polyethylenimine können Polymere mit zahlenmittleren Molekulargewichten von 300 bis 20000, bevorzugt 500 bis 10000, ganz besonders bevorzugt 500 bis 5000, eingesetzt werden. Das Gewichtsverhältnis zwischen eingesetztem Alkylenoxid und Polyethylenimin liegt im Bereich von 100 : 1 bis 0,1 : 1, bevorzugt im Bereich 50 : 1 bis 0,5 : 1, ganz besonders bevorzugt im Bereich 20 : 1 bis 0,5 : 1.

Für die Polymerisation in Gegenwart der Polyether b) seien.als Komponente a) folgende radikalisch polymerisierbare Monomere genannt:

Vinylester von aliphatischen, gesättigten oder ungesättigten C₁-C₂₄-Carbonsäuren, wie z.B. Ameisensäure, Essigsäure, Propionsäure, Buttersäure, Valeriansäure, Isovaleriansäure, Capronsäure, Caprylsäure, Caprinsäure, Undecylensäure, Laurinsäure, Myristinsäure, Palmitinsäure, Palmitoleinsäure, Stearinsäure, Ölsäure, Arachinsäure, Behensäure, Lignocerinsäure, Cerotinsäure sowie Melissensäure.

Bevorzugt werden Vinylester der oben genannten C₁-C₁₂-Carbonsäuren, insbesondere der C₁-C₆-Carbonsäuren, verwendet. Ganz besonders bevorzugt ist Vinylacetat.

Selbstverständlich können auch Mischungen der jeweiligen Monomeren aus der Gruppe a) copolymerisiert werden.

Die Vinylester (a) können daneben auch in Mischung mit einem oder mehreren, ethylenisch ungesättigten copolymerisierbaren Comonomeren (c) eingesetzt werden, wobei der Anteil dieser zusätzlichen Monomeren auf maximal 50 Gew.-% beschränkt sein sollte. Bevorzugt sind Anteile von 0 bis 20 Gew.-%. Der Begriff ethylenisch ungesättigt bedeutet, daß die Monomere zumindest eine radikalisch polymerisierbare Kohlenstoff-Kohlenstoff Doppelbindung besitzen, die mono-, di-, tri- oder tetrasubstituiert sein kann.

Die bevorzugten zusätzlich eingesetzten ethylenisch ungesättigten Comonomere (c) können durch die folgende allgemeine Formel beschrieben werden:

X-C (O) CR¹⁵=CHR¹⁴

wobei
X ausgewählt ist aus der Gruppe der Reste -OH, -OM, -OR¹⁶, NH₂, -NHR¹⁶, N(R¹⁶)₂ ;
M ist ein Kation ausgewählt aus der Gruppe bestehend aus: Na⁺, K⁺, Mg⁺⁺, Ca⁺⁺, Zn⁺⁺, NH₄⁺_{'} Alkylammonium, Dialkylammonium, Trialkylammonium und Tetraalkylammonium;
die Reste R¹⁶ können identisch oder verschieden ausgewählt werden aus der Gruppe bestehend aus -H, C₁-C₄₀ linear- oder verzweigtkettige Alkylreste, N,N-Dimethylaminoethyl, 2-Hydroxyethyl, 2-Methoxyethyl, 2-Ethoxyethyl, Hydroxypropyl, Methoxypropyl oder Ethoxypropyl.

R¹⁵ und R¹⁴ sind unabhängig voneinander ausgewählt aus der Gruppe bestehend aus: -H, C₁-C₈ linear- oder verzweigtkettige Alkylketten, Methoxy, Ethoxy, 2-Hydroxyethoxy, 2-Methoxyethoxy und 2-Ethoxyethyl.

Repräsentative aber nicht limitierende Beispiele von geeigneten Monomeren (c) sind zum Beispiel Acrylsäure oder Methacrylsäure und deren Salze, Ester und Amide. Die Salze können von jedem beliebigen nicht toxischen Metall, Ammonium oder substituierten Ammonium-Gegenionen abgeleitet sein.

Die Ester können abgeleitet sein von C₁-C₄₀ linearen, C₃-C₄₀ verzweigtkettigen oder C₃-C₄₀ carbocyclischen Alkoholen, von mehrfachfunktionellen Alkoholen mit 2 bis etwa 8 Hydroxylgruppen wie Ethylenglycol, Hexylenglycol, Glycerin und 1,2,6-Hexantriol, von Aminoalkoholen oder von Alkoholethern wie Methoxyethanol und Ethoxyethanol, (Alkyl)Polyethylenglykolen, (Alkyl)Polypropylenglykolen oder ethoxylierten Fettalkoholen, beispielsweise C₁₂-C₂₄-Fettalkoholen umgesetzt mit 1 bis 200 Ethylenoxid-Einheiten.

Ferner eignen sich N,N-Dialkylaminoalkylacrylate- und -methacrylate und N-Dialkylaminoalkylacryl- und -methacrylamide der allgemeinen Formel (III) 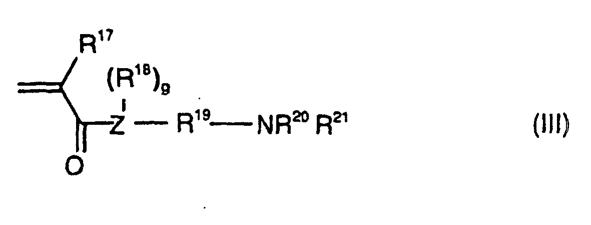
mit
R¹⁷ = H, Alkyl mit 1 bis 8 C-Atomen,
R¹⁸ = H, Methyl,
R¹⁹ = Alkylen mit 1 bis 24 C-Atomen, optional substituiert durch Alkyl,
R²⁰, R²¹ = C₁-C₄₀ Alkylrest,
Z = Stickstoff für g = 1 oder Sauerstoff für g = 0

Die Amide können unsubstituiert, N-Alkyl oder N-Alkylamino monosubstituiert oder N,N-dialkylsubstituiert oder N,N-dialkylaminodisubstituiert vorliegen, worin die Alkyl- oder Alkylaminogruppen von C₁-C₄₀ linearen, C₃-C₄₀ verzweigtkettigen, oder C₃-C₄₀ carbocyclischen Einheiten abgeleitet sind. Zusätzlich können die Alkylaminogruppen quaternisiert werden.

Bevorzugte Comonomere der Formel III sind N,N-Dimethylaminomethyl(meth)acrylat, N,N-Diethylaminomethyl(meth)acrylat, N,N-Dimethylaminoethyl(meth)acrylat, N,N-Diethylaminoethyl-(meth)acrylat, N-[3-(dimethylamino)propyl]methacrylamid und N-[3-(dimethylamino)propyl]acrylamid.

Ebenfalls verwendbare Comonomere (c) sind substituierte Acrylsäuren sowie Salze, Ester und Amide davon, wobei die Substituenten an den Kohlenstoffatomen in der zwei oder drei Position der Acrylsäure stehen, und unabhängig voneinander ausgewählt sind aus der Gruppe bestehend aus C₁-C₄ Alkyl, -CN, COOH besonders bevorzugt Methacrylsäure, Ethacrylsäure und 3-Cyanoacrylsäure. Diese Salze, Ester und Amide dieser substituierten Acrylsäuren können wie oben für die Salze, Ester und Amide der Acrylsäure beschrieben ausgewählt werden.

Andere geeignete Comonomere (c) sind Allylester von C₁-C₄₀ linearen, C₃-C₄₀ verzweigtkettigen oder C₃-C₄₀ carbocyclische Carbonsäuren, Vinyl- oder Allylhalogenide, bevorzugt Vinylchlorid und Allylchlorid, Vinylether, bevorzugt Methyl-, Ethyl-, Butyl-oder Dodecylvinylether, Vinylformamid, Vinylmethylacetamid, Vinylamin; Vinyllactame, bevorzugt Vinylpyrrolidon und Vinylcaprolactam, Vinyl- oder Allyl-substituierte heterocyclische Verbindungen, bevorzugt Vinylpyridin, Vinyloxazolin und Allylpyridin.

Weiterhin sind N-Vinylimidazole der allgemeinen Formel IV geeignet, worin R²² bis R²⁴ unabhängig voneinander für Wasserstoff, C₁-C₄-Alkyl oder Phenyl steht:

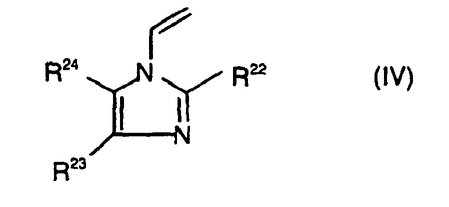

Weitere geeignete Comonomere (c) sind Diallylamine der allgemeinen Formel (V) 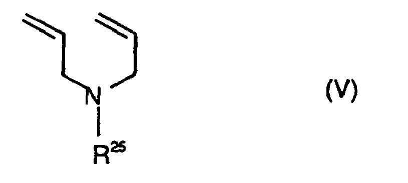 mit R²⁵ = C₁- bis C₂₄-Alkyl

Weitere geeignete Comonomere (c) sind Vinylidenchlorid; und Kohlenwasserstoffe mit mindestens einer Kohlenstoff-Kohlenstoff Doppelbindung, bevorzugt Styrol, alpha-Methylstyrol, tert.-Butylstyrol, Butadien, Isopren, Cyclohexadien, Ethylen, Propylen, 1-Buten, 2-Buten, Isobutylen, Vinyltoluol, sowie Mischungen dieser Monomere.

Besonders geeignete Comonomere (c) sind Acrylsäure, Methacrylsäure, Ethylacrylsäure, Methylacrylat, Ethylacrylat, Propylacrylat, n-Butylacrylat, iso-Butylacrylat, t-Butylacrylat, 2-Ethylhexylacrylat, Decylacrylat, Methylmethacrylat, Ethylmethacrylat, Propylmethacrylat, n-Butylmethacrylat, iso-Butylmethacrylat, t-Butylmethacrylat, 2-Ethylhexylmethacrylat, Decylmethacrylat, Methylethacrylat, Ethylethacrylat, n-Butylethacrylat, iso-Butylethacrylat, t-Butyl-ethacrylat, 2-Ethylhexylethacrylat, Decylethacrylat, Stearyl(meth)acrylat, 2,3-Dihydroxypropylacrylat, 2,3-Dihydroxypropylmethacrylat, 2-Hydroxyethylacrylat, Hydroxypropylacrylate, 2-Hydroxyethylmethacrylat, 2-Hydroxyethylethacrylat, 2-Methoxyethylacrylat, 2-Methoxyethylmethacrylat, 2-Methoxyethylethacrylat, 2-Ethoxyethylmethacrylat, 2-Ethoxyethylethacrylat, Hydroxypropylmethacrylate, Glycerylmonoacrylat, Glycerylmonomethacrylat, Polyalkylenglykol(meth)acrylate, ungesättigte Sulfonsäuren wie zum Beispiel Acrylamidopropansulfonsäure;

Acrylamid, Methacrylamid, Ethacrylamid, N-Methylacrylamid, N,N-Dimethylacrylamid, N-Ethylacrylamid, N-Isopropylacrylamid, N-Butylacrylamid, N-t-Butylacrylamid, N-Octylacrylamid, N-t-Octylacrylamid, *N*-Octadecylacrylamid, N-Phenylacrylamid, N-Methylmethacrylamid, N-Ethylmethacrylamid, N-Dodecylmethacrylamid, 1-Vinylimidazol, 1-Vinyl-2-methylvinylimidazol, N,N-Dimethylaminomethyl(meth)acrylat, N,N-Diethylaminomethyl(meth)-acrylat, N,N-Dimethylaminoethyl(meth)acrylat, N,N-Diethylaminoethyl(meth)acrylat, N,N-Dimethylaminobutyl(meth)acrylat, N,N-Diethylaminobutyl(meth)acrylat, N,N-Dimethylaminohexyl(meth)-acrylat, N,N-Dimethylaminooctyl(meth)acrylat, N,N-Dimethylaminododecyl(meth)acrylat, N-[3-(dimethylamino)propyl]methacrylamid, N-[3-(dimethylamino)propyl]acrylamid, N-[3-(dimethylamino)-butyl]methacrylamid, N-[8-(dimethylamino)octyl]methacrylamid, N-[12-(dimethylamino)dodecyl]methacrylamid, N-[3-(diethylamino)-propyl]methacrylamid, N-[3-(diethylamino)propyl]acrylamid;

Maleinsäure, Fumarsäure, Maleinsäureanhydrid und seine Halbester, Crotonsäure, Itaconsäure, Diallyldimethylammoniumchlorid, Vinylether (zum Beispiel: Methyl-, Ethyl-, Butyl- oder Dodecylvinylether), Vinylformamid, Vinylmethylacetamid, Vinylamin; Methylvinylketon, Maleimid, Vinylpyridin, Vinylimidazol, Vinylfuran, Styrol, Styrolsulfonat, Allylalkohol, und Mischungen daraus.

Von diesen sind besonders bevorzugt Acrylsäure, Methacrylsäure, Maleinsäure, Fumarsäure, Crotonsäure, Maleinsäureanhydrid sowie dessen Halbester, Methylacrylat, Methylmethacrylat, Ethylacrylat, Ethylmethacrylat, n-Butylacrylat, n-Butylmethacrylat, t-Butylacrylat, t-Butylmethacrylat, Isobutylacrylat, Isobutylmethacrylat, 2-Ethylhexylacrylat, Stearylacrylat, Stearylmethacrylat, N-t-Butylacrylamid, N-Octylacrylamid, 2-Hydroxyethylacrylat, Hydroxypropylacrylate, 2-Hydroxyethylmethacrylat, Hydroxypropylmethacrylate, Alkylenglykol(meth)acrylat-e, Styrol, ungesättigte Sulfonsäuren wie zum Beispiel Acrylamidopropansulfonsäure, Vinylpyrrolidon, Vinylcaprolactam, Vinylether (z.B.: Methyl-, Ethyl-, Butyl- oder Dodecylvinylether), Vinylformamid, Vinylmethylacetamid, Vinylamin, 1-Vinylimidazol, 1-Vinyl-2-methylimidazol, N,N-Dimethylaminomethylmethacrylat und N-[3-(dimethylamino)-propyl]methacrylamid; 3-Methyl-1-vinylimidazaliumchlorid, 3-Methyl-1-vinylimidazoliumanethylsulfat, N,N-Dimethylaminoethylmethacrylat, N-[3-(dimethylamino)propyl]methacrylamid quaternisiert mit Methylchlorid, Methylsulfat oder Diethylsulfat.

Monomere, mit einem basischen Stickstoffatom, können dabei auf folgende Weise quarternisiert werden:

Zur Quaternisierung der Amine eignen sich beispielsweise Alkylhalogenide mit 1 bis 24 C-Atomen in der Alkylgruppe, z.B. Methylchlorid, Methylbromid, Methyliodid, Ethylchlorid, Ethylbromid, Propylchlorid, Hexylchlorid, Dodecylchlorid, Laurylchlorid und Benzylhalogenide, insbesondere Benzylchlorid und Benzylbromid. Weitere geeignete Quaternierungsmittel sind Dialkylsulfate, insbesondere Dimethylsulfat oder Diethylsulfat. Die Quaternierung der basischen Amine kann auch mit Alkylenoxiden wie Ethylenoxid oder Propylenoxid in Gegenwart von Säuren durchgeführt werden. Bevorzugte Quaternierungsmittel sind: Methylchlorid, Dimethylsulfat oder Diethylsulfat.

Die Quaternisierung kann vor der Polymerisation oder nach der Polymerisation durchgeführt werden.

Außerdem können die Umsetzungsprodukte von ungesättigten Säuren, wie z.B. Acrylsäure oder Methacrylsäure, mit einem quaternisierten Epichlorhydrin der allgemeinen Formel (VI) eingesetzt werden (R²⁶ = C₁- bis C₄₀-Alkyl).

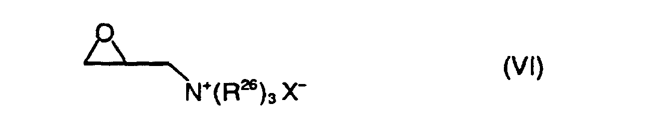

Beispiele hierfür sind zum Beispiel:
(Meth)acryloyloxyhydroxypropyltrimethylammoniumchlorid und (Meth)acryloyloxyhydroxypropyltriethylammoniumchlorid.

Die basischen Monomere können auch kationisiert werden, indem sie mit Mineralsäuren, wie z.B. Schwefelsäure, Chlorwasserstoffsäure, Bromwasserstoffsäure, Iodwasserstoffsäure, Phosphorsäure oder Salpetersäure, oder mit organischen Säuren, wie z.B. Ameisensäure, Essigsäure, Milchsäure, oder Citronensäure, neutralisiert werden.

Zusätzlich zu den oben genannten Comonomeren können als Comonomere (c) sogenannte Makromonomere wie zum Beispiel silikonhaltige Makromonomere mit ein oder mehreren radikalisch polymerisierbaren Gruppen oder Alkyloxazolinmakromonomere eingesetzt werden, wie sie zum Beispiel in der EP 408 311 beschrieben sind.

Des weiteren können fluorhaltige Monomere, wie sie beispielsweise in der EP 558423 beschrieben sind, vernetzend wirkende oder das Molekulargewicht regelnde Verbindungen in Kombination oder alleine eingesetzt werden.

Als Regler können die üblichen dem Fachmann bekannten Verbindungen, wie zum Beispiel Schwefelverbindungen (z.B.: Mercaptoethanol, 2-Ethylhexylthioglykolat, Thioglykolsäure oder Dodecylmercaptan), sowie Tribromchlormethan oder andere Verbindungen, die regelnd auf das Molekulargewicht der erhaltenen Polymerisate wirken, verwendet werden.

Es können gegebenenfalls auch thiolgruppenhaltige Silikonverbindungen eingesetzt werden.
Bevorzugt werden silikonfreie Regler eingesetzt.

Als vernetzende Monomere können Verbindungen mit mindestens zwei ethylenisch ungesättigten Doppelbindungen eingesetzt werden, wie zum Beispiel Ester von ethylenisch ungesättigten Carbonsäuren, wie Acrylsäure oder Methacrylsäure und mehrwertigen Alkoholen, Ether von mindestens zweiwertigen Alkoholen, wie zum Beispiel Vinylether oder Allylether.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglycolmonoester, 2,2-Bis(4-hydroxyphenyl)-propan, 2,2-Bis[4-(2-hydroxypropyl)-phenyl]propan, Diethylenglykol, Triethylenglykol, Tetraethylenglykol, Dipropylenglykol, Tripropylenglykol, Tetrapropylenglykol, 3-Thio-pentasi-1,5-diol, sowie Polyethylenglykole, Polypropylenglykole und Polytetrahydrofurane mit Molekulargewichten von jeweils 200 bis 10 000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer sind die Vinylester oder die Ester einwertiger, ungesättigter Alkohole mit ethylenisch ungesättigten C3- bis C6-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Vernetzer sind Ester ungesättigter Carbonsäuren mit den oben beschriebenen mehrwertigen Alkoholen, beispielsweise der Ölsäure, Crotonsäure, Zimtsäure oder 10-Undecensäure.

Außerdem geeignet sind geradkettige oder verzweigte, lineare oder cyclische aliphatische oder aromatische Kohlenwasserstoffe, die über mindestens zwei Doppelbindungen verfügen, welche bei den aliphatischen Kohlenwasserstoffen nicht konjugiert sein dürfen, z.B. Divinylbenzol, Divinyltoluol, 1,7-Octadien, 1,9-Decadien, 4-Vinyl-1-cyclohexen, Trivinylcyclohexen oder Polybutadiene mit Molekulargewichten von 200 bis 20000.

Ferner geeignet sind Amide von ungesättigten Carbonsäuren, wie z.B. Acryl- und Methacrylsäure, Itaconsäure, Maleinsäure und N-Allylaminen von mindestens zweiwertigen Aminen, wie z.B. 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin oder entsprechende Ammoniumsalze, z.B. Triallylmethylammoniumchlorid oder -methylsulfat, als Vernetzer geeignet.

Weiterhin können N-Vinylverbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z.B. N,N'-Divinylethylenharnstoff oder N,N'-Divinylpropylenharnstoff eingesetzt werden.

Weitere geeignete Vernetzer sind Divinyldioxan, Tetraallylsilan oder Tetravinylsilan.

Besonders bevorzugte Vernetzer sind beispielsweise Methylenbisacrylamid, Divinylbenzol, Triallylamin und Triallylammoniumsalze, Divinylimidazol, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind, sowie Allyl- oder Vinylether von mehrwertigen Alkoholen, beispielsweise 1,2-Ethandiol, 1,4-Butandiol, Diethylenglykol, Trimethylolpropan, Glycerin, Pentaerythrit, Sorbitan und Zucker wie Saccharose, Glucose, Mannose.

Ganz besonders bevorzugt als Vernetzer sind Pentaerythrittriallylether, Allylether von Zuckern wie Saccharose, Glucose, Mannose, Divinylbenzol, Methylenbisacrylamid, N,N'-Divinylethylenharnstoff, und (Meth-)Acrylsäureester von Glykol, Butandiol, Trimethylolpropan oder Glycerin oder (Meth)Acrylsäureester von mit Ethylenoxid und/oder Epichlorhydrin umgesetzten Glykol, Butandiol, Trimethylolpropan oder Glycerin.

Der Anteil der vernetzend wirkenden Monomeren beträgt 0 bis 10 Gew.-%, bevorzugt 0,1 bis 5 Gew.-%, ganz besonders bevorzugt 0,2 bis 2 Gew.-%.

Bei der Polymerisation zur Herstellung der erfindungsgemäßen Polymerisate können gegebenenfalls auch andere Polymere, wie zum Beispiel Polyamide, Polyurethane, Polyester, Homo- und Copolymere von ethylenisch ungesättigten Monomeren, zugegen sein. Beispiele für solche zum Teil auch in der Kosmetik eingesetzten Polymeren sind die unter den Handelsnamen bekannten Polymere Amerhold™, Ultrahold™, Ultrahold Strong™, Luviflex™ VBM, Luvimer™, Acronal™, Acudyne™, Stepanhold™, Lovocryl™, Versatyl™, Amphomer™ oder Eastma AQ™.

Die erfindungsgemäßen Comonomere (c) können, sofern sie ionisierbare Gruppen enthalten, vor oder nach der Polymerisation, zum Teil oder vollständig mit Säuren oder Basen neutralisiert werden, um so zum Beispiel die Wasserlöslichkeit oder -dispergierbarkeit auf ein gewünschtes Maß einzustellen.

Als Neutralisationsmittel für Säuregruppen tragende Monomere können zum Beispiel Mineralbasen wie Natriumcarbonat, Alkalihydroxide sowie Ammoniak, organische Basen wie Aminoalkohole speziell 2-Amino-2-Methyl-1-Propanol, Monoethanolamin, Diethanolamin, Triethanolamin, Triisopropanolamin, Tri[(2-hydroxy)1-Propyl]amin, 2-Amino-2-Methyl-1,3-Propandiol, 2-Amino-2-hydroxymethyl-1,3-Propandiol sowie Diamine, wie zum Beispiel Lysin, verwendet werden.

Zur Herstellung der Polymerisate können die Monomeren der Komponente a) in Gegenwart der Polyether sowohl mit Hilfe von Radikale bildenden Initiatoren als auch durch Einwirkung energiereicher Strahlung, worunter auch die Einwirkung energiereicher Elektronen verstanden werden soll, polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azo-bis-isobutyronitril, Azo-bis-(2-amidinopropan)dihydrochlorid oder 2-2'-Azo-bis-(2-methyl-butyronitril). Geeignet sind auch Initiatormischungen oder Redox-Initiator-Systeme, wie z.B. Ascorbinsäure/Eisen(II)sulfat/-Natriumperoxodisulfat, tert.-Butylhydroperoxid/Natriumdisulfit, tert.-Butylhydroperoxid/Natriumhydroxymethansulfinat. Bevorzugt werden organische Peroxide eingesetzt.

Die verwendeten Mengen an Initiator bzw. Initiatorgemischen bezogen auf eingesetztes Monomer liegen zwischen 0,01 und 10 Gew.-%, vorzugsweise zwischen 0,1 und 5 Gew.-%.

Die Polymerisation erfolgt im Temperaturbereich von 40 bis 200°C, bevorzugt im Bereich von 50 bis 140°C, besonders bevorzugt im Bereich von 60 bis 110°C. Sie wird üblicherweise unter atmosphärischem Druck durchgeführt, kann jedoch auch unter vermindertem oder erhöhtem Druck, vorzugsweise zwischen 1 und 5 bar, ablaufen.

Die Polymerisation kann beispielsweise als Lösungspolymerisation, Polymerisation in Substanz, Emulsionspolymerisation, umgekehrte Emulsionspolymerisation, Suspensionspolymerisation, umgekehrte Suspensionspolymerisation oder Fällungspolymerisation durchgeführt werden, ohne daß die verwendbaren Methoden darauf beschränkt sind.

Bei der Polymerisation in Substanz kann man so vorgehen, daß man die polyetherhaltige Verbindung b) in mindestens einem Monomer der Gruppe a) und eventuell weiteren Comonomeren der Gruppe c) löst und nach Zugabe eines Polymerisationsinitiators die Mischung auspolymerisiert. Die Polymerisation kann auch halbkontinuierlich durchgeführt werden, indem man zunächst einen Teil, z.B. 10 % des zu polymerisierenden Gemisches aus der polyetherhaltigen Verbindung b), mindestens einem Monomeren der Gruppe a), eventuell weiteren Comonomeren der Gruppe c) und Initiator vorlegt, das Gemisch auf Polymerisationstemperatur erhitzt und nach dem Anspringen der Polymerisation den Rest der zu polymerisierenden Mischung nach Fortschritt der Polymerisation zugibt. Die Polymerisate können auch dadurch erhalten werden, daß man die polyetherhaltigen Verbindungen der Gruppe b) in einem Reaktor vorlegt, auf die Polymerisationstemperatur erwärmt und mindestens ein Monomer der Gruppe a), eventuell weiteren Comonomeren der Gruppe c) und Polymerisationsinitiator entweder auf einmal, absatzweise oder vorzugsweise kontinuierlich zufügt und polymerisiert.

Falls gewünscht, kann die oben beschriebene Polymerisation auch in einem Lösemittel durchgeführt werden. Geeignete Lösemittel sind beispielsweise Alkohole, wie Methanol, Ethanol, n-Propanol, Isopropanol, n-Butanol, sek.-Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Glycerin und Dioxan. Die Polymerisation kann auch in Wasser als Lösemittel durchgeführt werden. In diesem Fall liegt zunächst eine Lösung vor, die in Abhängigkeit von der Menge der zugegebenen Monomeren der Komponente a) in Wasser mehr oder weniger gut löslich ist. Um wasserunlösliche Produkte, die während der Polymerisation entstehen können, in Lösung zu überführen, kann man beispielsweise organische Lösemittel zusetzen, wie einwertige Alkohole mit 1 bis 3 Kohlenstoffatomen, Aceton oder Dimethylformamid. Man kann jedoch auch bei der Polymerisation in Wasser so verfahren, daß man die wasserunlöslichen Polymerisate durch Zugabe üblicher Emulgatoren oder Schutzkolloide, z.B. Polyvinylalkohol, in eine feinteilige Dispersion überführt.

Als Emulgatoren verwendet man beispielsweise ionische oder nichtionische Tenside, deren HLB-Wert im Bereich von 3 bis 13 liegt. Zur Definition des HLB-Werts wird auf die Veröffentlichung von W.C. Griffin, J. Soc. Cosmetic Chem., Band 5, 249 (1954), hingewiesen.

Die Menge an Tensiden, bezogen auf das Polymerisat, beträgt 0,1 bis 10 Gew.-%. Bei Verwendung von Wasser als Lösemittel erhält man Lösungen bzw. Dispersionen der Polymerisate. Sofern man Lösungen des Polymerisates in einem organischen Lösemittel herstellt bzw. in Mischungen aus einem organischen Lösemittel und Wasser, so verwendet man pro 100 Gew.-Teile des Polymerisates 5 bis 2000, vorzugsweise 10 bis 500 Gew.-Teile des organischen Lösemittels oder des Lösemittelgemisches.

Bevorzugt sind Polymere, die erhältlich sind durch radikalische Polymerisation von

| | | |
|---|---|---|
| a) | 10 bis 98 Gew.-% | mindestens eines Vinylesters von C₁-C₂₄-Carbonsäuren in Gegenwart von |
| b) | 2 bis 90 Gew.-% | mindestens einer polyetherhaltigen Verbindung und |
| c) | 0 bis 50 Gew.-% | eines oder mehreren weiteren copolymerisierbaren Monomeren |

Besonders bevorzugt sind Polymere, die erhältlich sind durch radikalische Polymerisation von

| | | |
|---|---|---|
| a) | 50 bis 97 Gew.-% | mindestens eines Vinylesters von C₁-C₂₄-Carbonsäuren in Gegenwart von |
| b) | 3 bis 50 Gew.-% | mindestens einer polyetherhaltigen Verbindung und |
| c) | 0 bis 30 Gew.-% | eines oder mehreren weiteren copolymerisierbaren Monomeren |

Ganz besonders bevorzugt sind Polymere, die erhältlich sind durch radikalische Polymerisation von

| | | |
|---|---|---|
| a) | 60 bis 97 Gew.-% | mindestens eines Vinylesters von C₁-C₂₄-Carbonsäuren in Gegenwart von |
| b) | 3 bis 40 Gew.-% | mindestens einer polyetherhaltigen Verbindung und |
| c) | 0 bis 20 Gew.-% | eines oder mehreren weiteren copolymerisierbaren Monomeren |

Die erhaltenen Polymerisate können auch nachträglich vernetzt werden, indem man die Hydroxylgruppen bzw. Aminogruppen im Polymer mit mindestens bifunktionellen Reagentien umsetzt. Bei niedrigen Vernetzungsgraden erhält man wasserlösliche Produkte, bei hohen Vernetzungsgrade wasserquellbare bzw. unlösliche Produkte.

Beispielsweise können die erfindungsgemäßen Polymerisate mit Aldehyden, Dialdehyden, Ketonen und Diketonen, z.B. Formaldehyd, Acetaldehyd, Glyoxal, Glutaraldehyd, Succindialdehyd oder Terephthalaldehyd, umgesetzt werden. Desweiteren eignen sich aliphatische oder aromatische Carbonsäuren, beispielsweise Maleinsäure, Oxalsäure, Malonsäure, Succinsäure oder Citronensäure, bzw. Carbonsäurederivaten wie Carbonsäureester, -anhydride oder -halogenide. Ferner sind mehrfunktionelle Epoxide geeignet, z.B. Epichlorhydrin, Glycidylmethacrylat, Ethylenglykoldiglycidylether, 1,4-Butandioldiglycidylether oder 1,4-Bis-(glycidyloxy)benzol. Ferner eigenen sich Diisocyanate, beispielsweise Hexamethylendiisocyanat, Isophorondiisocyanat, Methylendiphenyldiisocyanat, Toluyiendiisocyanat oder Divinylsulfon.

Weiterhin eignen sich anorganische Verbindungen wie Borsäure oder Borsäuresalze, die im folgenden zusammenfassend als Borate bezeichnet werden, beispielsweise Natriumanetaborat, Borax (Dinatriumtetraborat), sowie Salze mehrwertiger Kationen, z.B. Kupfer(II)salze wie Kupfer(II)acetat oder Zink-, Aluminium-, Titansalze.

Borsäure bzw. Borsäuresalze wie Natriummetaborat oder Dinatriumtetraborat eignen sich bevorzugt zur nachträglichen Vernetzung. Dabei können die Borsäure bzw. Borsäuresalze, bevorzugt als Salzlösungen, den Lösungen der erfindungsgemäßen Polymerisate zugegeben werden. Bevorzugt werden die Borsäure bzw. Borsäuresalze den wässerigen Polymerisatlösungen hinzugefügt.

Die Borsäure bzw. Borsäuresalze können den Polymerlösungen direkt nach der Herstellung zugefügt werden. Es ist aber auch möglich, die Borsäure bzw. Borsäuresalze nachträglich den kosmetischen Formulierungen mit den erfindungsgemäßen Polymerisaten zuzusetzen, bzw. während des Herstellungsprozesses der kosmetischen Formulierungen.

Der Anteil Borsäure bzw. Borsäuresalze bezogen auf die erfindungsgemäßen Polymere beträgt 0 bis 15 Gew-%, bevorzugt 0,1 bis 10 Gew.-%, besonders bevorzugt 0,5 bis 5 Gew.-%.

Die Polymerisatlösungen und -dispersionen können durch verschiedene Trocknungsverfahren wie z.B. Sprühtrocknung, Fluidized Spray Drying, Walzentrocknung oder Gefriertrocknung in Pulverform überführt werden. Als Trocknungsverfahren wird bevorzugt die Sprühtrocknung eingesetzt. Aus dem so erhaltenen Polymer-Trockenpulver läßt sich durch Lösen bzw. Redispergieren in Wasser erneut eine wäßrige Lösung bzw. Dispersion herstellen. Die Überführung in Pulverform hat den Vorteil einer besseren Lagerfähigkeit, einer einfacheren Transportmöglichkeit sowie eine geringere Neigung für Keimbefall.

Anstelle der wasserdampfdestillierten Polymerlösungen können auch die alkoholischen Polymerlösungen direkt in Pulverform überführt werden.

Die erfindungsgemäßen wasserlöslichen oder wasserdispergierbaren Polyalkylenoxid- bzw. Polyglycerin-haltigen Polymerisate eignen sich hervorragend zur Verwendung in haarkosmetischen Formulierungen.

Die erfindungsgemäßen Polymere, hergestellt durch radikalische Polymerisation von Vinylestern und optional weiterer polymerisierbarer Monomerer in Gegenwart polyetherhaltiger Verbindungen eignen sich als Stylingmittel und/oder Konditioniermittel in haarkosmetischen Zubereitungen wie Haarkuren, Haarlotionen, Haarspülungen, Haaremulsionen, Spitzenfluids, Egalisierungsmittel für Dauerwellen, "Hot-Oil-Treatment"-Präparate, Conditioner, Festigerlotionen oder Haarsprays. Je nach Anwendungsgebiet können die haarkosmetischen Zubereitungen als Spray, Schaum, Gel, Gelspray oder Mousse appliziert werden.

Die erfindungsgemäßen haarkosmetischen Formulierungen enthalten in einer bevorzugten Ausführungsform

| | | |
|---|---|---|
| a) | 0,05 bis 20 Gew.-% | des erfindungsgemäßen Polymeren, hergestellt durch radikalische Polymerisation von Vinylestern und optional weiterer polymerisierbarer Monomerer in Gegenwart polyetherhaltiger Verbindungen, |
| b) | 20 bis 99,95 Gew.-% | Wasser und/oder Alkohol |
| c) | 0 bis 79,50 Gew.-% | weitere Bestandteile |

Unter Alkohol sind alle in der Kosmetik üblichen Alkohole zu verstehen, z.B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d.h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z.B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Stabilisatoren, pH-Wert-Regulatoren, Farbstoffe, Viskositätsregulierer, Gelbildner, Salze, Feuchthaltemittel, Rückfetter und weitere übliche Additive.

Weiterhin zählen hierzu alle in der Kosmetik bekannten Styling-und Conditionerpolymere, die in Kombination mit den erfindungsgemäßen Polymerisaten eingesetzt werden können, falls ganz spezielle Eigenschaften eingestellt werden sollen.

Als herkömmliche Haarkosmetik-Polymere eignen sich beispielsweise anionische Polymere. Solche anionischen Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane (Luviset® P.U.R.) und Polyharnstoffe. Besonders geeignete Polymere sind Copolymere aus t-Butylacrylat, Ethylacrylat, Methacrylsäure (z.B. Luvimer® 100P), Copolymere aus N-tert.-Butylacrylamid, Ethylacrylat, Acrylsäure (Ultrahold® 8, strong), Copolymere aus Vinylacetat, Crotonsäure und gegebenenfall weiteren Vinylestern (z.B. Luviset® Marken), Maleinsäureanhydridcopolymere, ggf. mit Alkoholen umgesetzt, anionische Polysiloxane, z.B. carboxyfunktionelle, Copolymere aus Vinylpyrrolidon, t-Butylacrylat, Methacrylsäure (z.B Luviskol® VBM).

Ganz besonders bevorzugt werden als anionische Polymere Acrylate mit einer Säurezahl größer gleich 120 und Copolymere aus t-Butylacrylat, Ethylacrylat, Methacrylsäure.

Weitere geeignete Haarkosmetik-Polymere sind kationische Polymere mit der Bezeichnung Polyquaternium nach INCI, z.B. Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam N-Vinylpyrrolidon/N-Vinylimidazoliumsalzen (Luviquat® Hold); kationische Cellulosederivate (Polyquaternium-4 und -10), Acrylamidcopolymere (Polyquaternium-7).

Als weitere Haarkosmetik-Polymere sind auch neutrale Polymere geeignet wie Polyvinylpyrrolidone, Copolymere aus N-Vinylpyrrolidon und Vinylacetat und/oder Vinylpropionat, Polysiloxane, Polyvinylcaprolactam und Copolymere mit N-Vinylpyrrolidon, Polyethylenimine und deren Salze, Polyvinylamine und deren Salze, Cellulosederivate, Polyasparaginsäuresalze und Derivate.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen Polymere eignen sich insbesondere als Festigungsmittel in Haarstyling-Zubereitungen, insbesondere Haarsprays (Aerosolsprays und Pumpsprays ohne Treibgas) und Haarschäume (Aerosolschäume und Pumpschäume ohne Treibgas). In einer bevorzugten Ausführungsform enthalten diese Zubereitungen

| | | |
|---|---|---|
| a) | 0,1 bis 10 Gew.-%' | des erfindungsgemäßen Polymeren, hergestellt durch radikalische Polymerisation von Vinylestern und optional weiterer polymerisierbarer Monomerer in Gegenwart polyetherhaltiger Verbindungen |
| b) | 20 bis 99,9 Gew.-% | Wasser und/oder Alkohol |
| c) | 0 bis 70 Gew.-% | eines Treibmittel |
| d) | 0 bis 20 Gew.-% | weitere Bestandteile |

Treibmittel sind die für Haarsprays oder Aerosolschäume üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.

Eine erfindungsgemäß bevorzugte Formulierung für Aerosolhaarschäume enthält

| | | |
|---|---|---|
| a) | 0,1 bis 10 Gew.-% | des erfindungsgemäßen Polymeren, hergestellt durch radikalische Polymerisation von Vinylestern und optional weiterer polymerisierbarer Monomerer in Gegenwart polyetherhaltiger Verbindungen |
| b) | 55 bis 94,8 Gew.-% | Wasser und/oder Alkohol |
| c) | 5 bis 20 Gew.-% | eines Treibmittel |
| d) | 0,1 bis 5 Gew.-% | eines Emulgators |
| e) | 0 bis 10 Gew.-% | weitere Bestandteile |

Als Emulgatoren können alle in Haarschäumen üblicherweise eingesetzten Emulgatoren verwendet werden. Geeignete Emulgatoren können nichtionisch, kationisch bzw. anionisch sein.

Beispiele für nichtionische Emulgatoren (INCI-Nomenklatur) sind Laurethe, z.B. Laureth-4; Cetethe, z.B. Cetheth-1, Polyethylenglycolcetylether; Cetearethe, z.B. Cetheareth-25, Polyglycolfettsäureglyceride, hydroxyliertes Lecithin, Lactylester von Fettsäuren, Alkylpolyglycoside.
Beispiele für kationische Emulgatoren sind Cetyldimethyl-2-hydroxyethylammoniumdihydrogenphosphat, Cetyltrimoniumchlorid, Cetyltrimoniumbromid, Cocotrimoniummethylsulfat, Quaternium-1 bis x (INCI).
Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein:

| | | |
|---|---|---|
| a) | 0,1 bis 10 Gew.-% | des erfindungsgemäßen Polymeren, hergestellt durch radikalische Polymerisation von Vinylestern und optional weiterer polymerisierbarer Monomerer in Gegenwart polyetherhaltiger Verbindungen |
| b) | 60 bis 99,85 Gew.-% | Wasser und/oder Alkohol |
| c) | 0,05 bis 10 Gew.-% | eines Gelbildners |
| d) | 0 bis 20 Gew.-% | weitere Bestandteile |

Als Gelbildner können alle in der Kosmetik üblichen Gelbildner eingesetzt werden. Hierzu zählen leicht vernetzte Polyacrylsäure, beispielsweise Carbomer (INCI), Cellulosederivate, z.B. Hydroxypropylcellulose, Hydroxyethylcellulose, kationisch modifizierte Cellulosen, Polysaccharide, z.B. Xanthum Gummi, Caprylic/Capric Triglyceride, Sodium acrylates Copolymer, Polyquaternium-32 (and) Paraffinum Liquidum (INCI), Sodium Acrylates Copolymer (and) Paraffinum Liquidum (and) PPG-1 Trideceth-6, Acrylamidopropyl Trimonium Chloride/Acrylamide Copolymer, Steareth-10 Allyl Ether Acrylates Copolymer, Polyquaternium-37 (and) Paraffinum Liquidum (and) PPG-1 Trideceth-6, Polyquaternium 37 (and) Propylene Glycol Dicaprate Dicaprylate (and) PPG-1 Trideceth-6, Polyquaternium-7, Polyquaternium-44.

Die erfindungsgemäßen Polymere können auch in Shampooformulierungen als Festigungs- und/oder Konditioniermittel eingesetzt werden. Als Konditioniermittel eignen sich insbesondere Polymere...mit kationischer Ladung. Bevorzugte Shampooformulierungen enthalten

| | |
|---|---|
| a) 0,05 bis 10 Gew.-% | des erfindungsgemäßen Polymeren, hergestellt durch radikalische Polymerisation von Vinylestern und optional weiterer polymerisierbarer Monomerer in Gegenwart polyetherhaltiger Verbindungen |
| b) 25 bis 94,95 Gew.-% | Wasser |
| c) 5 bis 50 Gew.-% | Tenside |
| c) 0 bis 5 Gew.-% | eines weiteren Konditioniermittels |
| d) 0 bis 10 Gew.-% | weitere kosmetische Bestandteile |

In den Shampooformulierungen können alle in Shampoos üblicherweise eingesetzte anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlauroylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt weden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z.B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den erfindungsgemäßen Polymeren eingesetzt werden. Hierzu zählen beispielsweise kationische Polymere mit der Bezeichnung Polyquaternium nach INCI, insbesondere Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidcopolymere (Polyquaternium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA).

### Herstellungsbeispiele:

### Herstellvorschrift für Beispiele 1 bis 32

In einem Polymerisationsgefäß wird die polyetherhaltige Verbindung vorgelegt und unter Rühren und leichtem Stickstoffstrom auf 80°C erhitzt. Unter Rühren werden Vinylacetat und gegebenenfalls die weiteren Monomeren in 3 h zudosiert. Gleichzeitig wird eine Lösung von 1,4 g tert.-Butylperpivalat in 30 g Methanol ebenfalls in 3 h zugegeben. Danach wird noch 2 h bei 80°C gerührt.Nach dem Abkühlen wird das Polymerisat in 450 ml Methanol gelöst. Nach dem anschließenden Lösungsmittelaustausch durch Wasserdampfdestillation erhält man eine wäßrige Lösung bzw. Dispersion.

Die K-Werte wurden 1%ig in Ethanol bestimmt.

**Tabelle**

| Beispiel | Pfropfgrundlage | Vinylester | Comonomer | K-Wert |
|---|---|---|---|---|
| 1*) | PEG 1500¹ 72 g | Vinylacetat, 410 g | - | 55 |
| 2*) | PEG 4000 72 g | Vinylacetat, 410 g | - | 59 |
| 3*) | PEG 6000, 273 g | Vinylacetat, 410 g | - | 47 |
| 4*) | PEG 6000, 137 g | Vinylacetat, 410 g | | 53 |
| 5*) | PEG 6000, 615 g | Vinylacetat 410g | - | 44 |
| 6*) | PEG 6000, 410 g | Vinylacetat 410 g | | 47 |
| 7*) | PEG 9000, 137 g | Vinylacetat, 410 g | - | 60 |
| 8*) | Polyglycerin 2200, 72 g | Vinylacetat, 410 g | - | 56 |
| 9*) | PEG-PPG-Blockcopolymer 8000², 72 g | Vinylacetat, 410g | - | 49 45 |
| 10*) | Methylpolyethylenglykol 2000³ 72 g | Vinylacetat, 410 g | - | 47 |
| 11*) | Alkylpolyethylenglykol 3500⁴ 72 g | Vinylacetat, 410 g | - | 50 |
| 12*) | PPG 4000⁵ 72 | Vinylacetat 410 g | | 51 |
| 13*) | PEG 20000 72 g | Vinylacetat, 410 g | - | 72 |
| 14*) | PEG 20000 410 g | Vinylacetat, 410 g | - | 57 |
| 15*) | PEG 20000 137 g | Vinylacetat, 410 g | - | 62 |
| 16*) | PEG 20000 615 g | Vinylacetat, 410 g | - | 59 |
| 17*) | PEG 35000 615 g | Vinylacetat, 410 g | - | 69 |
| 18*) | PEG 35000 137g | Vinylacetat, 410 g | - | 85 85 |
| 19*) | PEG 35000 205 g | Vinylacetat, 410 g | - | 72 |
| 20*) | Dimethicone copolyol⁶, 202 g | Vinylacetat, 410g | - | 62 |
| 21*) | Poly(Natriummethacrylat-co-methyl- polyethylenglykolmethacrylat)⁷ 103 g | Vinylacetat, 410g | | 48 |
| 22 | ethoxyliertes Polyethylenimin⁸ 279 g | Vinylacetat, 410 g | | 56 |
| 23*) | PEG 6000, 72 g | Vinylacetat, 386 g | Methylmethacrylat, 24 g | 52 52 |
| 24*) | PEG 20000, 72 g | Vinylacetat, 205 g | N-Vinylpyrrolidon, 205 g | 64 |
| 25*) | PEG 20000, 72 g | Vinylacetat, 362 g | 3-Methyl-1-vinylimidazoliummethylsulfat, 48 g | 54 |
| 26*) | PEG 6000, 72 g | Vinylacetat, 164g | N-Vinylformamid, 246 g | 62 |
| 27*) | PEG 6000, 72g | Vinylacetat, 326 g | N-Vinylformamid, 82 g | 70 70 |
| 28*) | PEG 35000, 270g | Vinylacetat, 410 g | | 63 63 |
| 29*) | PEG 35000, 270g | Vinylacetat, 410 g | Pentaerythrittriallyl-ether, 1,6g | 76 |
| 30*) | PEG 35000, 270 g | Vinylacetat, 410 g | Pentaerythrittriallyl-ether, 0,8 g | 70 70 |
| 31*) | PEG 35000, 270 g | Vinylacetat, 410 g | N,N'-Divinyl-ethylenharnstoff 0,7 g | 78 |
| 32*) | PEG 12000, 270 g | Vinylacetat, 410 g | Pentaerythrittriallyl-ether, 1,6 g | 55 |

| | | | | |
|---|---|---|---|---|
| *)nicht erfindungsgemäß | | | | |
| ¹ PEG x: Polyethylenglykol mit mittlerem Molekulargewicht x (Gewichtsmittel) | | | | |
| ² Lutrol F 68 der Fa. BASF Aktiengesellschaft (PPG: Polypropylenglykol) | | | | |
| ³ Pluriol A 2000 E der Fa. BASF Aktiengesellschaft | | | | |
| ⁴ Lutensol AT 80 der Fa. BASF Aktiengesellschaft (C₁₆-C₁₈-Fettalkohol + 80 EO) | | | | |
| ⁵ Polypropylenglykol mit mittlerem Molekulargewicht 4000 | | | | |
| ⁶ Belsil DMC 6031TM der Fa. Wacker Chemie GmbH | | | | |
| ⁷ Molverhältnis Natriummethacrylat/Methylpolyethylenglykolmetbacrylat 4:1; Methylpolyethylenglykol mit Molmasse ca. 1000 | | | | |
| ⁸ hergestellt aus 12,5 % Polyethylenimin (mittleres Molekulargewicht 1400) und 87,5 % Ethylenoxid | | | | |

### Formulierungsbeispiele:

### Beispiel 33*): Formulierung Aerosolhaarschaum:

2,00 % Copolymer aus Beispiel 3
2,00 % Luviquat Mono LS (Coco trimonium methyl sulfat)
67,7 % Wasser
10,0 % Propan/Butan 3,5 bar (20°C)
q.s. Parfümöl

### Beispiel 34 (Vergleichsbeispiel):

2,00 % Polymergehalt Luviquat Hold (Polquaternium-46)
2,00 % Luviquat Mono LS (Coco trimonium methyl sulfat)
67,7 % Wasser
10,0 % Propan/Butan 3,5 bar (20°C)
q.s. Parfümöl

Mit Beispiel 33 und Beispiel 34 (Vergleichbeispiel) wurden Halbseitentests an Modellköpfen durchgeführt. Die Beurteilung erfolgte subjektiv durch geschulte Friseure und Labormitarbeiter.

### Notenskala von 1 (sehr gut) bis 3 (schwach)

| | Beispiel 33 | Beispiel 34 (Vergleichsbeispiel) |
|---|---|---|
| Aufschäumen: | 1 | 1 |
| Konsistenz des Schaumes: | 1 | 1 |
| Verteilbarkeit: | 1 | 1 |
| Griff nasses Haar: | 1- | 2 |
| Naßkämmbarkeit: | 1- | 2+ |
| Festigung: | 1 | 2+ |
| Trockenkämmbarkeit: | 2+ | 2. |
| Klebrigkeit: | 1 | 1- |
| Griff des trockenenHaares: | 1- | 2+ |
| Elastizität des Haares | 1 | 2- |

Die Formulierung aus Beispiel 33 bewirkte im Vergleich zur Formulierung aus Beispiel 34 (Vergleichsbeispiel) eine bessere Festigung, eine bessere Naßkämmbarkeit, eine geringere Klebrigkeit sowie eine erhöhte Elastizität der Haare.

### Beispiel 35*): Aerosolhaarschaum:

| | INCI |
|---|---|
| 4,00 % Copolymer aus Beispiel 17 | |
| 0,20 % Cremophor A 25 | Ceteareth-25 |
| 1,00 % Luviquat Mono CP | Hydroxyethyl cetyldimonium phosphate |
| 5,00 % Ethanol | |
| 1,00 % Panthenol | |
| 10,0 % Propan/Butan 3,5 bar (20°C) | |
| q.s. Parfümöl | |
| ad 100 % Wasser | |

### Beispiel 36*): Pumpschaum:

| | INCI |
|---|---|
| 2,00 % Copolymer aus Beispiel 26 | |
| 2,00 % Luviflex Soft (Polymergehalt) | |
| 1,20 % 2-Amino-2-methyl-1-propanol | |
| 0,20 % Cremophor A 25 | |
| 0,10 % Uvinul P 25 | PEG-25 PABA |
| q.s. Konservierungsmittel | |
| q.s. Parfümöl | |
| ad 100 % Wasser | |

### Beispiel 37*): Pumpspray

| | INCI |
|---|---|
| 4,00 % Copolymer aus Beispiel 24 | |
| 1,00 % Panthenol | |
| 0,10 % Uvinul MS 40 | Benzophenone-4 |
| q.s. Konservierungsmittel | |
| q.s. Parfümöl | |
| ad 100 % Wasser | |

### Beispiel 38*): Pumpspray:

| | INCI |
|---|---|
| 4,00 % Copolymer aus Beispiel 14 | |
| 1,00 % Panthenol | |
| 0,10 % Uvinul M 40 | Benzophenone-3 |
| q.s. Konservierungsmittel | |
| q.s. Parfümöl | |
| ad 100 % Ethanol | |

### Beispiel 39*): Haarspray:

| | INCI |
|---|---|
| 5,00 % Copolymer aus Beispiel 23 | |
| 0,10 % Siliconöl Dow Corning DC 190 | Dimethicone Copolyol |
| 35,00 % Dimethylether | |
| 5,00 % n-Pentan | |
| ad 100 % Ethanol | |
| q.s. Parfümöl | |

### Beispiel 40*): Haarspray VOC 55 %:

| | INCI |
|---|---|
| 3,00 % Copolymer aus Beispiel 4 | |
| 7,00 % Luviset P.U.R. | Polyurethane-1 |
| 40,00 % Dimethylether | |
| 15,00 % Ethanol | |
| q.s. Parfümöl | |
| ad 100 % Wasser | |

### Beispiel 41*): Haargel:

| | INCI |
|---|---|
| 0,50 % Carbopol 980 | Carbomer |
| 3,00 % Copolymer aus Beispiel 27 | |
| 0,10 % Phythantriol | |
| 0,50 % Panthenol | |
| q.s. Parfümöl | |
| q.s Konservierungsmittel | |
| ad 100 % Wasser | |

### Beispiel 42*): Haarshampoo bzw. Duschgel

| | INCI |
|---|---|
| 0,50 % Copolymer aus Beispiel 25 | |
| 40,00 % Texapon NSO | Sodium Laureth Sulfate |
| 5,00 % Tego Betain L 7 | Cocamidopropyl Betaine |
| 5,00 % Plantacare 2000 | Decyl Glucoside |
| 1,00 % Propylenglycol | |
| q.s. Citronensäure | |
| q.s. Konservierungsmittel | |
| 1,00 % Natriumchlorid | |
| ad 100 % Wasser | |

| | |
|---|---|
| *) nicht erfindungsgemäß | |

## Patentansprüche

1. Verwendung von Polymerisaten, die erhältlich sind durch radikalische Polymerisation von
a) mindestens einem Vinylester von C₁-C₂₄-Carbonsäuren in Gegenwart von
b) polyetherhaltigen Verbindungen erhältlich durch Umsetzung von Polyethyleniminen mit Alkylenoxiden
und
c) gegebenenfalls eines oder mehrerer weiterer copolymerisierbarer Monomeren
in haarkosmetischen Formulierungen.

2. Verwendung von Polymerisaten nach Anspruch 1, **dadurch gekennzeichnet, daß** als Alkylenoxide Ethylenoxid, Propylenoxid, Butylenoxid und Mischungen aus diesen verwendet werden.

3. Verwendung von Polymerisaten nach Ansprüchen i und 2, **dadurch gekennzeichnet, daß** als Alkylenoxid Ethylenoxid verwendet wird.

4. Verwendung von Polymerisaten nach Ansprüchen 1, 2 und 3, **dadurch gekennzeichnet, daß** das Polyethylenimin ein Molekulargewicht zwischen 300 und 20000 bsitzt.

5. Verwendung von Polymerisaten nach Anspruch 1, **dadurch gekennzeichnet, daß** c) ausgewählt wird aus der Gruppe:
Acrylsäure, Methacrylsäure, Maleinsäure, Fumarsäure, Crotonsäure, Maleinsäureanhydrid sowie dessen Halbester, Methylacrylat, Methylmethacrylat, Ethylacrylat, Ethylmethacrylat, n-Butylacrylat, n-Butylmethacrylat, t-Butylacrylat, t-Butylmethacrylat, Isobutylacrylat, Isobutylmethacrylat, 2-Ethylhexylacrylat, Stearylacrylat, Stearylmethacrylat, N-t-Butylacrylamid, N-Octylacrylamid, 2-Hydroxyethylacrylat, Hydroxypropylacrylate, 2-Hydroxyethylmethacrylat, Hydroxypropylmethacrylate, Alkylenglykol(meth)acrylate, Styrol, ungesättigte Sulfonsäuren wie z.B. Acrylamidopropansulfonsäure, Vinylpyrrolidon, Vinylcaprolactam, Vinylether (z.B.: Methyl-, Ethyl-, Butyl- oder Dodecylvinylether), Vinylformamid, Vinylmethylacetämid, Vinylamin, 1-Vinylimidazol, 1-Vinyl-2-methylimidazol, N,N-Dimethylaminomethylmethacrylat und N-[3-(dimethylamino)propyl]methacrylamid; 3-Methyl-1-vinylimidazoliumchlorid, 3-Methyl-1-vinylimidazoliummethylsulfat, N,N-Dimethylaminoethylmethacrylat, N-[3-(dimethylamino)-propyl]methacrylamid quaternisiert mit Methylchlorid, Methylsulfat oder Diethylsulfat.

6. Verwendung von Polymerisaten nach Anspruch 1, **dadurch gekennzeichnet, daß** die Mengenverhältnisse
a) 10 bis 90 Gew.-%
b) 2 bis 90 Gew.-%
c) 0 bis 50 Gew.-%
betragen.

7. Haarkosmetische Formulierungen, **dadurch gekennzeichnet, daß** sie wie folgt zusammengesetzt sind:
a) 0,05 bis 20 Gew.-% des Polymeren gemäß Anspruch 1
b) 20 bis 99,95 Gew.-% Wasser und/oder Alkohol
c) 0 bis 79,05 Gew.-% weitere Bestandteile

## Claims

1. The use of polymers which are obtainable by free-radical polymerization of
a) at least one vinyl ester of C₁-C₂₄-carboxylic acids in the presence of
b) polyether-containing compounds obtainable by reaction of polyethyleneimines with alkylene oxides
and
c) optionally one or more further copolymerizable monomer
in hair cosmetic formulations.

2. The use of polymers as claimed in claim 1, wherein the alkylene oxides are ethylene oxide, propylene oxide, butylene oxide and mixtures thereof.

3. The use of polymers as claimed in claims 1 and 2, wherein the alkylene oxide is ethylene oxide.

4. The use of polymers as claimed in claims 1, 2 and 3, wherein the polyethyleneimine has a molecular weight between 300 and 20000.

5. The use of polymers as claimed in claim 1, wherein c) is chosen from the group:
acrylic acid, methacrylic acid, maleic acid, fumaric acid, crotonic acid, maleic anhydride and half-esters thereof, methyl acrylate, methyl methacrylate, ethyl acrylate, ethyl methacrylate, n-butyl acrylate, n-butyl methacrylate, t-butyl acrylate, t-butyl methacrylate, isobutyl acrylate, isobutyl methacrylate, 2-ethylhexyl acrylate, stearyl acrylate, stearyl methacrylate, N-t-butylacrylamide, N-octylacrylamide, 2-hydroxyethyl acrylate, hydroxypropyl acrylates, 2-hydroxyethyl methacrylate, hydroxypropyl methacrylates, alkylene glycol (meth)acrylates, styrene, unsaturated sulfonic acids such as, for example, acrylamidopropane sulfonic acid, vinyl pyrrolidone, vinyl caprolactam, vinyl ethers, (e.g. methyl, ethyl, butyl or dodecyl vinyl ethers), vinylformamide, vinylmethylacetamide, vinylamine, 1-vinylimidazole, 1-vinyl-2-methylimidazole, N,N-dimethylaminomethyl methacrylate and N-[3-(dimethylamino)propyl]methacrylamide; 3-methyl-1-vinylimidazolium chloride, 3-methyl-1-vinylimidazolium methylsulfate, N,N-dimethylaminoethyl methacrylate, N-[3-(dimethylamino)propyl]methacrylamide quaternized with methyl chloride, methyl sulfate or diethyl sulfate.

6. The use of polymers as claimed in claim 1, wherein the quantitative ratios are
a) 10 to 90% by weight
b) 2 to 90% by weight
c) 0 to 50% by weight.

7. A hair cosmetic formulation which has the following composition:
a) 0.05 to 20% by weight of the polymer as in claim 1
b) 20 to 99.95% by weight of water and/or alcohol
c) 0 to 79.05% by weight of further constituents.

## Revendications

1. Utilisation de polymères que l'on peut obtenir par polymérisation radicalaire
a) d'au moins un ester vinylique d'acides carboxyliques en C₁-C₂₄ en présence
b) de composés contenant du polyéther que l'on peut obtenir par réaction de polyéthylèneimines avec des oxydes d'alkylène, et
c) éventuellement d'un ou de plusieurs autres monomères copolymérisables,
dans des formulations cosmétiques pour le cheveux.

2. Utilisation de polymères suivant la revendication 1, **caractérisée en ce que**, comme oxydes d'alkylène, on utilise de l'oxyde d'éthylène, de l'oxyde de propylène, de l'oxyde de butylène et des mélanges de ces derniers.

3. Utilisation de polymères suivant les revendications 1 et 2, **caractérisée en ce que**, comme oxyde d'alkylène, on utilise de l'oxyde d'éthylène.

4. Utilisation de polymères suivant les revendications 1, 2 et 3, **caractérisée en ce que** la polyéthylèneimine a un poids moléculaire entre 300 et 20.000.

5. Utilisation de polymères suivant la revendication 1, **caractérisée en ce que** c) est choisi parmi le groupe :
acide acrylique, acide méthacrylique, acide maléique, acide fumarique, acide crotonique, anhydride maléique ainsi que son hémiester, acrylate de méthyle, méthacrylate de méthyle, acrylate d'éthyle, méthacrylate d'éthyle, acrylate de n-butyle, méthacrylate de n-butyle, acrylate de t-butyle, méthacrylate de t-butyle, acrylate d'isobutyle, méthacrylate d'isobutyle, acrylate de 2-éthylhexyle, acrylate de stéaryle, méthacrylate de stéaryle, n-t-butylacrylamide, N-octylacrylamide, acrylate de 2-hydroxyéthyle, acrylates d'hydroxypropyle, méthacrylate de 2-hydroxyéthyle, méthacrylates d'hydroxypropyle, (méth)acrylates d'alkylèneglycol, styrène, acides sulfoniques insaturés, comme par exemple de l'acide acrylamidopropanesulfonique, vinylpyrrolidone, vinylcaprolactame, éther vinylique (par exemple : éther méthylvinylique, éthylvinylique, butylvinylique ou dodécylvinylique), vinylformamide, vinylméthylacétamide, vinylamine, 1-vinylimidazole, 1-vinyl-2-méthylimidazole, méthacrylate de N,N-diméthylaminométhyle et N-[3-(diméthylamino)propyl]-méthacrylamide, chlorure de 3-méthyl-1-vinylimidazolium, méthylsulfate de 3-méthyl-1-vinylimidazolium, méthacrylate de N,N-diméthylaminoéthyle, N-[3-(diméthylamino)-propyl]méthacrylamide quaternisé avec du chlorure de méthyle, du sulfate de méthyle ou du sulfate de diéthyle.

6. Utilisation de polymères suivant la revendication 1, **caractérisée en ce que** les proportions quantitatives sont
a) 10 à 90 % en poids,
b) 2 à 90 % en poids,
c) 0 à 50 % en poids.

7. Formulations cosmétiques pour les cheveux, **caractérisées en ce qu'**elles sont composées de la manière suivante :
a) 0,05 à 20 % en poids du polymère suivant la revendication 1,
b) 20 à 99,95 % en poids d'eau et/ou d'alcool,
c) 0 à 79,05% en poids d'autres éléments.
